# EUROPÄISCHE PATENTSCHRIFT

(11) **EP 1 179 988 B1**
(45) Veröffentlichungstag und Bekanntmachung des Hinweises auf die Patenterteilung: **10.03.2004**
(21) Anmeldenummer: 00940256.1
(22) Anmeldetag: 18.05.2000
(51) Int. Cl.: A61B 1/267

(54) **LARYNGOSKOP**
LARYNGOSCOPE
LARYNGOSCOPE

(30) Priorität: 21.05.1999 DE 19923334; 16.11.1999 DE 19955180
(43) Veröffentlichungstag der Anmeldung: 20.02.2002
(73) Patentinhaber: Karl Storz GmbH & Co. KG, 78532 Tuttlingen (DE)
(72) Erfinder: RUDISCHHAUSER, Jürgen, D-78532 Tuttlingen (DE); RENNER, Martin, D-78576 Liptingen (DE); IRION, Klaus, M., D-78576 Liptingen (DE); SCHWARZ, Peter, D-78532 Tuttlingen (DE); WEHRSTEIN, Helmut, D-78532 Tuttlingen (DE); KOCHER, Mark, D-71065 Sindelfingen (DE)
(74) Vertreter: Witte, Alexander, Dr.-Ing.
(86) Internationale Anmeldenummer: PCT/EP2000/004514
(87) Internationale Veröffentlichungsnummer: WO 2000/071018

(56) Entgegenhaltungen:
- EP-A- 0 901 772
- WO-A-98/19589
- DE-B- 1 766 713
- US-A- 4 320 745
- US-A- 4 877 016
- US-A- 5 827 178

## Beschreibung

Die vorliegende Erfindung betrifft ein Laryngoskop, mit einem Handgriff und mit einem im wesentlichen quer dazu angeordneten Spatel, der über eine Kupplung lösbar an dem Handgriff befestigt ist, ferner mit einem Lichtleiter und einem Bildleiter, die fest an dem Spatel angeordnet sind, wobei der Lichtleiter ein proximales Ende mit einer Lichteintrittsöffnung und wobei der Bildleiter ein proximales Ende mit einer Bildaustrittsöffnung aufweist, wobei die Lichteintrittsöffnung und die Bildaustrittsöffnung im Bereich der Kupplung angeordnet sind und wobei der Handgriff im Bereich der Kupplung eine Lichtaustrittsöffnung sowie eine Bildeintrittsöffnung aufweist, die ein Einkoppeln des Lichtsignals von dem Handgriff in den Lichtleiter sowie ein Auskoppeln des Bildsignals aus dem Bildleiter ermöglichen, ferner mit einem Zentrierelement, das die Bildeintrittsöffnung und die Bildaustrittsöffnung paßgenau zueinander zentriert.

Ein derartiges Laryngoskop ist aus der EP 0 901 772 A1 bekannt.

Bei dem bekannten Laryngoskop, das auch als Video-Laryngoskop bezeichnet werden kann, dient der Bildleiter dazu, ein Bild im Bereich des distalen Endes des Spatels, d.h. aus dem Rachenraum eines Patienten, aufzunehmen und an eine Bildwiedergabeeinheit zu übertragen. Die Bildwiedergabeeinheit kann, sie muß jedoch nicht an dem Handgriff befestigt sein. In jedem Fall muß ein aufgenommenes Bildsignal jedoch über die lösbare Kupplung hinweg transportiert werden. Dies geschieht durch Überkoppeln des Bildsignals aus dem am Spatel angeordneten Bildleiter in die Bildeintrittsöffnung eines im Handgriff angeordneten Bildaufnahmesystems.

Das Ein- und Auskoppeln eines Bildsignals von einem Bildleiter in einen anderen ist stets mit Qualitätsverlusten verbunden. Wie sich gezeigt hat, sind diese um so geringer, je exakter die beiden gegenüberstehenden Öffnungen zueinander ausgerichtet sind. Das aus der EP 0 901 772 A1 bekannte Laryngoskop weist einen Rastverschluß im Bereich der Kupplung auf, der gewährleistet, daß der Laryngoskopspatel derart an eine im Handgriff angeordnete Bildaufnahmeeinheit konnektierbar ist, daß der für den behandelnden Arzt wichtige Bereich des Rachenraums stets abgebildet werden kann.

Aus der US 5,846,186 und der US 5,800,344 sind Video-Laryngoskope bekannt, bei denen der Bildleiter im Unterschied zu dem eingangs genannten Laryngoskop nicht über die Kupplung geführt ist. Hierdurch werden Qualitätsverluste vermieden, da der Bildleiter einteilig ausgeführt werden kann. Bei diesen Laryngoskopen ist allerdings durch die Kabel im proximalen Bereich des Spatels die Handhabung für den Arzt erschwert.

Aus der US 5,827,178 ist ein weiteres Video-Laryngoskop bekannt. Das Problem der Überkopplung der Bildsignale vom Spatel auf den Handgriff ist hierin jedoch nicht angesprochen.

Es ist eine Aufgabe der vorliegenden Erfindung, ein Laryngoskop der eingangs genannten Art bereitzustellen, das hinsichtlich seiner mechanischen Eigenschaften kompatibel zu einer Vielzahl von vorhandenen Laryngoskopen ist und das gleichzeitig eine stabile Kopplung zwischen dem Handgriff und dem Spatel gewährleistet.

Diese Aufgabe wird bei dem eingangs genannten Laryngoskop dadurch gelöst, daß die Kupplung eine Standardkupplung zum Verbinden von Laryngoskop-Spateln mit Handgriffen gemäß der Internationalen Norm 7376-3 ist und daß die Kupplung am proximalen Ende des Bildleiters einen zusätzlichen Kupplungsbereich aufweist, durch den die Kontaktfläche der Kupplung gegenüber der Norm ISO 7376-3 erweitert wird und in dem die Kopplung für den Bildleiter angeordnet ist.

Durch das Zentrierelement wird nicht nur eine einmal vorhandene Ausrichtung der Koppelöffnungen fixiert, sondern es ist darüber hinaus auch die absolute Position der Koppelöffnungen zueinander gewährleistet. Technisch gesehen wird also nicht nur verhindert, daß sich die Positionen der Koppelöffnungen nach dem Zusammenstecken von Spatel und Handgriff verändern, sondern es wird darüber hinaus schon beim Zusammenstecken von Handgriff und Spatel eine optimale Justierung der Koppelöffnungen erreicht. Auf diese Weise stehen die Bildeintritts- und die Bildaustrittsöffnung stets optimal zueinander, so daß Qualitätsverluste beim Überkoppeln des Bildsignals minimal sind.

Die genannte Norm definiert eine Standardkupplung für das Verbinden von Spateln und Handgriffen bei Laryngoskopen. Dementsprechend sind zahlreiche Laryngoskope mit dieser Standardkupplung im Einsatz. Die Standardkupplung besitzt den Vorteil, daß die Spatel und Handgriffe der bereits verwendeten Laryngoskope wahlweise auch mit dem Spatel und dem Handgriff des erfindungsgemäßen Laryngoskops kombiniert werden können, wenngleich in diesem Fall unter Umständen die Bildwiedergabeeinheit nicht genutzt werden kann. In einer Notfallsituation besteht jedoch aufgrund dieser Maßnahme die Möglichkeit, einen hinsichtlich seiner Größe besser geeigneten Spatel mit dem Handgriff des erfindungsgemäßen Laryngoskops zu kombinieren. Insgesamt wird aufgrund der genannten Maßnahmen somit die Verwendungsbreite erhöht.

Bei dem erfindungsgemäßen Laryngoskop weist die Kupplung an dem proximalen Ende des Bildleiters einen Kupplungsbereich auf, der außerhalb eines durch die internationale Norm ISO 7376-3 definierten Kupplungsbereichs liegt.

Alternativ zu dieser Maßnahme ist es grundsätzlich möglich, das proximale Ende des Bildleiters innerhalb der durch die Norm ISO 7376-3 vorgegebenen Abmessungen in die Kupplung zu integrieren. Die erfindungsgemäße Maßnahme besitzt demgegenüber den Vorteil, daß der durch die Norm definierte Bereich nicht verändert werden muß, was die Einhaltung der Norm wesentlich erleichtert. Darüber hinaus wird aufgrund dieser Maßnahme ein zweiter Kupplungsbereich bereitgestellt, der die Stabilität und den Halt der Kupplung verbessert. Dies ist insbesondere im Hinblick auf die Paßgenauigkeiten, mit denen die einander zugeordneten Eintrittsund Austrittsöffnungen positioniert werden müssen, vorteilhaft.

Gleichzeitig bietet das erfindungsgemäße Laryngoskop eine ebenso einfache Handhabung wie das eingangs genannte Laryngoskop. Die genannte Aufgabe ist daher vollständig gelöst.

In einer Ausgestaltung der Erfindung zentriert das Zentrierelement die Bildeintrittsöffnung und die Bildaustrittsöffnung mechanisch zueinander.

Diese Maßnahme gewährleistet eine einfache und robuste Handhabung, insbesondere beim Zusammenfügen des Spatels mit dem Handgriff.

In einer weiteren Ausgestaltung zentriert das Zentrierelement die Bildeintrittsöffnung und die Bildaustrittsöffnung sowohl in radialer als auch in axialer Richtung zueinander.

Diese Maßnahme ist besonders vorteilhaft im Hinblick darauf, daß nicht nur eine radiale Verschiebung der einander zugeordneten Öffnungen, sondern auch eine axiale Verschiebung eine Verschlechterung der Bildqualität zur Folge haben kann. Zur Erreichung einer optimalen Bildqualität ist es daher von Vorteil, die einander zugeordneten Öffnungen in jeder Richtung zu zentrieren.

In einer weiteren Ausgestaltung der zuvor genannten Maßnahmen fixiert das Zentrierelement die Eintritts- und Austrittsöffnungen mit einer Paßungenauigkeit von weniger als 0,5 mm, vorzugsweise weniger als 0,1 mm.

Dies Größenordnung hat sich bei praktischen Versuchen als vorteilhaft erwiesen, um eine gleichbleibende Bildqualität auch bei einer rauhen und mit Krafteinwirkungen verbundenen Anwendung des Laryngoskops, insbesondere in einer Notfallsituation, zu gewährleisten.

In einer weiteren Ausgestaltung weist das Zentrierelement zumindest einen Konus sowie einen entsprechenden Gegenkonus auf, von denen einer an dem Handgriff und der andere an dem Spatel angeordnet ist.

Eine derartige Ausgestaltung hat sich für das Zentrierelement als besonders vorteilhaft erwiesen, da sie einerseits einfach und robust ist, während sie andererseits die zuvor genannten vorteilhaften Maßnahmen in sich vereint.

In einer weiteren Ausgestaltung weist das Laryngoskop eine elektronische Bildzentriereinheit auf.

Eine elektronische Bildzentriereinheit läßt sich insbesondere dadurch realisieren, daß in dem Handgriff des Laryngoskops ein elektronischer Bildaufnehmer, beispielsweise ein CCD-Chip, angeordnet wird, dessen lichtempfindliche, aktive Fläche größer ist, als die eigentlich benötigte Fläche. In diesem Fall ist der elektronische Bildaufnehmer in der Lage, das von dem Bildleiter übertragene Bildsignal auch dann aufzunehmen, wenn die Justierung der Bildeintritts- und Austrittsöffnung nicht mehr exakt eingehalten ist. Mit an sich bekannten Maßnahmen der elektronischen Bildverarbeitung kann dann der "richtige" Bildausschnitt extrahiert werden. Die Maßnahme besitzt sowohl für sich genommen als auch in Ergänzung zu einem mechanischen Zentrierelement den Vorteil, daß die Bildqualität des erfindungsgemäßen Laryngoskops auch bei Belastungen und Krafteinwirkungen gleichmäßig hoch gehalten werden kann.

In einer weiteren, besonders bevorzugten Ausgestaltung der Erfindung sind die Bildeintrittsöffnung und die Lichtaustrittsöffnung in unterschiedlichen, axial zueinander versetzten Koppelebenen angeordnet.

Diese Maßnahme besitzt den Vorteil, daß hierdurch auf einfache Weise ein Einstreuen des Lichtsignals in den Bildleiter verhindert ist, wodurch die Bildqualität des erfindungsgemäßen Laryngoskops nochmals verbessert ist.

In einer weiteren Ausgestaltung der Erfindung weist das Laryngoskop eine an dem Handgriff angeordnete Bildwiedergabeeinheit zum Wiedergeben eines aufgenommenen Bildes auf.

Durch diese Maßnahme wird das erfindungsgemäße Laryngoskop autonom, d.h. es kann unabhängig von einem externen Monitor und anderen externen Geräten verwendet werden. Hierdurch wird die Handhabung und der Aufwand insbesondere im Hinblick auf Notfallsituationen beträchtlich vereinfacht.

In einer weiteren Ausgestaltung der zuvor genannten Maßnahme ist die Bildwiedergabeeinheit auf einer vom distalen Ende des Spatels abgewandten Seite des Handgriffs angeordnet.

Diese Maßnahme besitzt den Vorteil, daß der behandelnde Arzt das von der Bildwiedergabeeinheit gelieferte Bild praktisch von hinten, also von der Rückseite des Laryngoskops her betrachten kann. Dies ist besonders günstig, da der behandelnde Arzt somit schnell zwischen dem von der Bildwiedergabeeinheit gelieferten Bild und einem direkten Blick in den Rachenraum des Patienten wechseln kann, ohne seinen Kopf stark bewegen zu müssen.

In einer weiteren Ausgestaltung ist die Bildwiedergabeeinheit um eine Längsachse des Handgriffs drehbar.

Diese Maßnahme besitzt den Vorteil, daß der behandelnde Arzt die Ausrichtung der Bildwiedergabeeinheit seinen Bedürfnissen leicht anpassen kann. Dabei läßt sich eine Drehung der Bildwiedergabeeinheit um die Längsachse des Handgriffs robuster realisieren, als eine Drehung um eine zur Längsachse des Handgriffs orthogonale Achse. Das erfindungsgemäße Laryngoskop dieser Ausgestaltung ist daher trotz der gewonnenen Verstellmöglichkeit sehr robust.

In einer weiteren Ausgestaltung ist die Bildwiedergabeeinheit in bezug auf die Längsachse des Handgriffs kippbar.

Auch diese Maßnahme besitzt den Vorteil, daß der behandelnde Arzt die Ausrichtung der Bildwiedergabeeinheit seinen Bedürfnissen bei der Verwendung des Laryngoskops anpassen kann.

In einer weiteren Ausgestaltung ist die Bildwiedergabeeinheit von dem Handgriff abnehmbar.

Diese Maßnahme besitzt den Vorteil, daß die Bildwiedergabeeinheit im Fall einer Beschädigung auf einfache Weise ausgetauscht werden kann. Ein weiterer Vorteil ist, daß der behandelnde Arzt die Bildwiedergabeeinheit vom Handgriff abnehmen kann, wenn er sie bei einer Behandlung nicht benötigt. In diesem Fall entspricht das erfindungsgemäße Laryngoskop hinsichtlich seiner Abmessungen und seiner Handhabbarkeit jedem herkömmlichen Laryngoskop ohne Bildwiedergabeeinheit.

In einer weiteren Ausgestaltung besteht zumindest ein Teil des Spatels aus einem lichtleitenden Material, welches den Lichtleiter bildet.

Diese Maßnahme besitzt den Vorteil, daß auf zusätzliche optische Fasern für den Lichtleiter verzichtet werden kann. Hierdurch läßt sich die Robustheit des Laryngoskops nochmals verbessern, während gleichzeitig Kosten bei der Herstellung eingespart werden können. Hinzu kommt, daß der Lichtaustritt zur Beleuchtung des Rachenraums auf einfache Weise am distalen Ende des Spatels angeordnet werden kann, ohne daß die funktionsbedingte Formgebung des Spatels verändert werden muß.

In einer weiteren Ausgestaltung weist das Laryngoskop mindestens zwei Bildleiter sowie zwei Bilderfassungseinheiten auf.

Diese Maßnahme besitzt den Vorteil, daß sich ein stereoskopisches und damit räumliches Bild erzeugen läßt, was dem behandelnden Arzt die Orientierung beim Intubieren eines Patienten nochmals erleichtert.

In einer weiteren Ausgestaltung ist am distalen Ende des Spatels ein Gassensor zur Messung von Parametern eines Gasgemisches angeordnet.

Vorzugsweise dient der Gassensor zum Bestimmen eines Sauerstoff- und/oder CO₂-Gehaltes. Die Maßnahme ist besonders vorteilhaft, wenn das Laryngoskop in Notfallsituationen verwendet wird, um einen erstickenden Patienten zu intubieren. In diesem Fall kann nämlich auf einfache Weise die Sauerstoffmenge im Bereich des Luftröhreneingangs bestimmt werden.

In einer weiteren Ausgestaltung der zuvor genannten Maßnahme ist der Gassensor mit einer im Handgriff angeordneten Auswerteeinheit verbunden.

Diese Maßnahme besitzt den Vorteil, daß die vergleichsweise empfindliche Auswerteeinheit in dem Laryngoskop geschützt untergebracht ist. Gleichzeitig bietet das Laryngoskop so die Möglichkeit, die Signale des Gassensors ohne externe Geräte zu verwenden.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: ein Laryngoskop mit einem Licht- und einem Bildleiter in einer Seitenansicht,
- Fig. 2: das Laryngoskop aus Fig. 1 in einer perspektivischen Darstellung, wobei der Spatel von dem Handgriff abgetrennt ist,
- Fig. 3: das Laryngoskop aus Fig. 1 entlang der Schnittlinie III-III,
- Fig. 4: die Kupplung des Larnygoskops aus Fig. 3 in einer Detailansicht,
- Fig. 5: die Kupplung aus Fig. 4, wobei der Spatel und der Handgriff von einander getrennt sind,
- Fig. 6: ein Ausführungsbeispiel eines erfindungsgemäßen Laryngoskops in einer Seitenansicht,
- Fig. 7: ein weiteres Ausführungsbeispiel eines erfindungsgemäßen Laryngoskops
- Fig. 8: ein autonomes Videolaryngoskop, und
- Fig. 9: ein zweites Beispiel eines autonomen Videolaryngoskops.

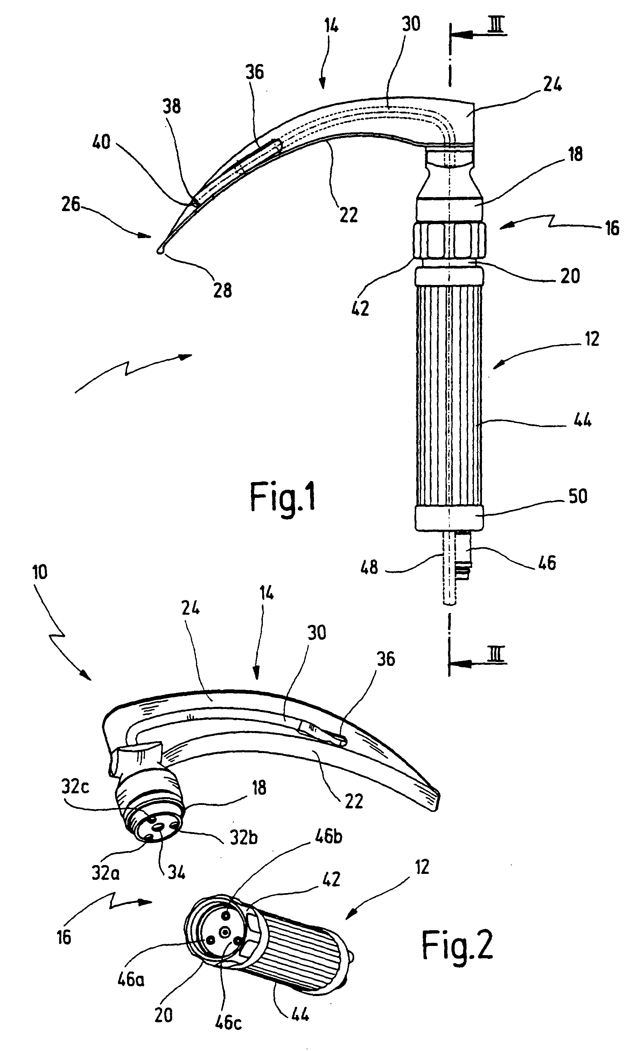

In Fig. 1 bis 3 ist ein Laryngoskop in seiner Gesamtheit mit der Bezugsziffer 10 bezeichnet. Das Laryngoskop 10 fällt nicht unter den Schutz der hier beanspruchten Erfindung. Die folgende Beschreibung dient jedoch deren ergänzender Erläuterung.

Das Laryngoskop 10 besitzt einen Handgriff 12 sowie einen Spatel 14, die über eine Kupplung 16 miteinander verbunden sind. Der spatelseitige Teil der Kupplung 16 ist mit der Bezugsziffer 18 und der griffseitige Teil mit der Bezugsziffer 20 bezeichnet.

Der Spatel 14 besitzt in an sich bekannter Weise ein Spatelblatt 22, von dem aus eine seitliche Wand 24 im wesentlichen senkrecht nach oben steht. An seinem distalen Ende 26 weist das Spatelblatt 22 eine Wulst 28 auf. Auf der dem Spatelblatt 22 abgewandten Seite der seitlichen Wand 24 ist ein Rohr 30 verlegt, das sowohl einen Lichtleiter 32 als auch einen Bildleiter 34 beherbergt. Der Lichtleiter 32 besteht im vorliegenden Ausführungsbeispiel aus drei einzelnen Lichtleitersträngen 32a, 32b, 32c, die radial um den Bildleiter 34 herum angeordnet sind, wie dies an der geöffneten Kupplung 16 in Fig. 2 erkennbar ist.

Das Rohr 30 erstreckt sich auf der Rückseite der seitlichen Wand 24 bis zu einem Langloch 36 und tritt dort durch die seitliche Wand 24 hindurch auf das Spatelblatt 22. Am distalen Ende des Rohrs 30 weist der Lichtleiter 32 eine Lichtaustrittsöffnung 38 auf. Ebenso besitzt der Bildleiter 34 dort eine Bildeintrittsöffnung 40. Die gemeinsame Anordnung des Lichtleiters 32 und des Bildleiters 34 in einem Rohr 30 ist an sich im Stand der Technik bekannt und braucht daher hier nicht ausführlicher dargestellt zu werden.

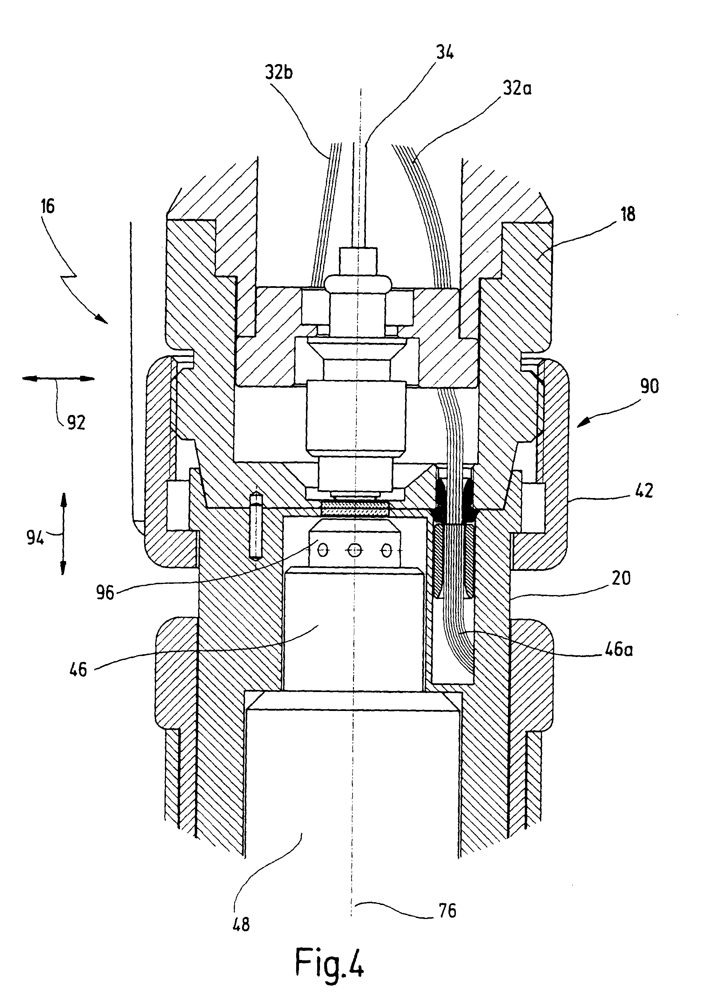

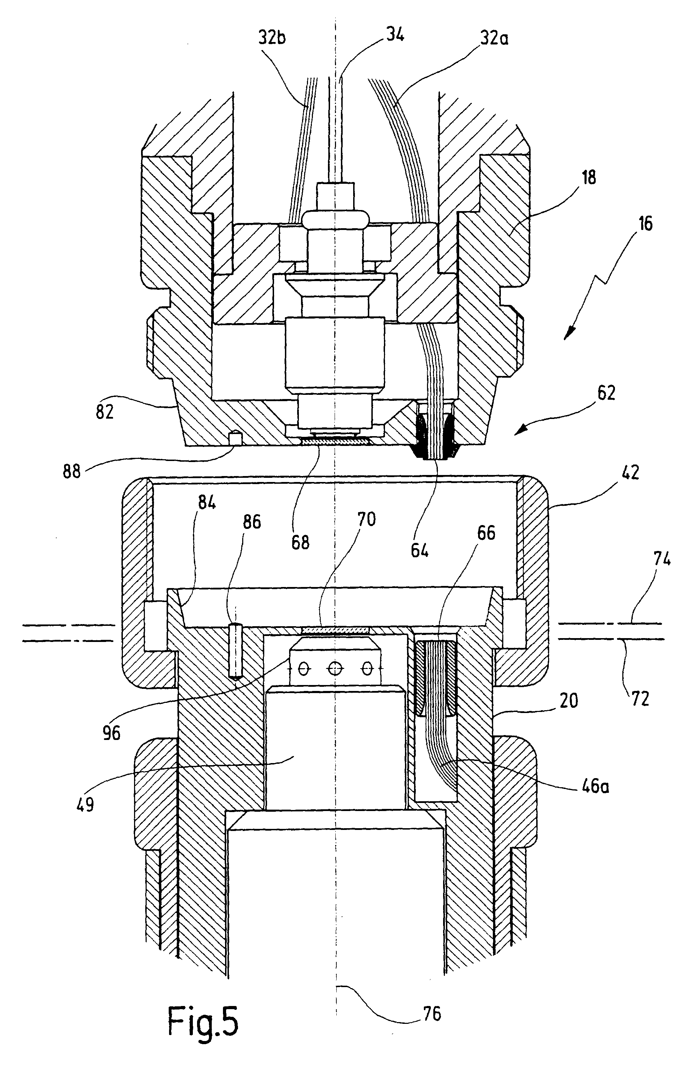

Die Kupplung 16 des Laryngoskops 10 ist eine bajonettartige Kupplung, bei der die miteinander zu verbindenden Teile 18, 20 durch eine Haltemutter 42 fixiert werden. Der weitere Aufbau der Kupplung wird nachfolgend anhand der Fig. 4 und 5 detaillierter beschrieben.

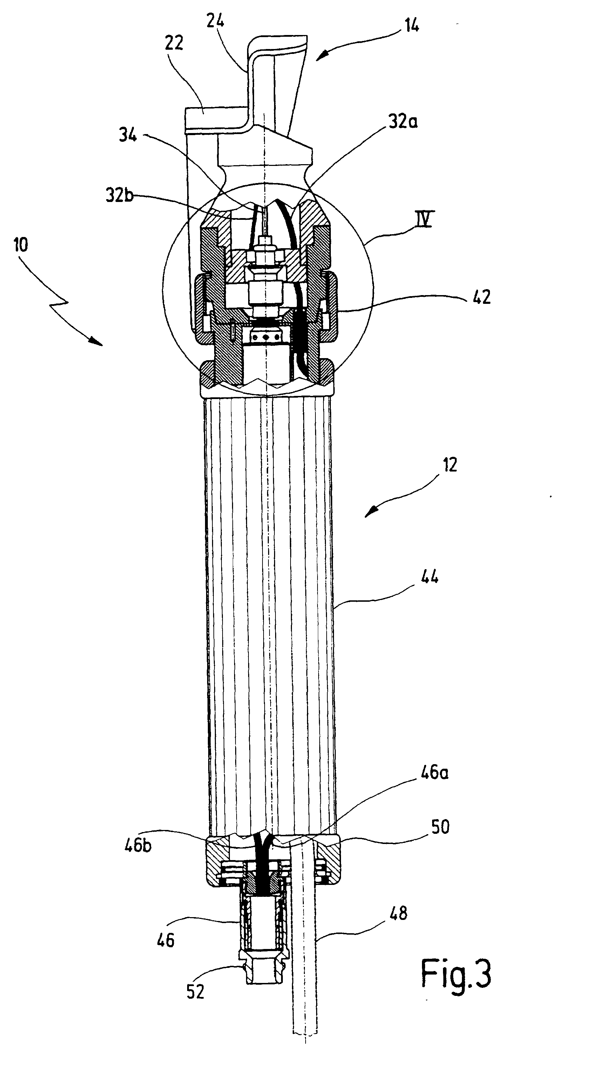

Der Handgriff 12 des Laryngoskops 10 weist in an sich bekannter Weise einen rohrförmigen Schaft 44 auf, in dem weitere Bestandteile des Laryngoskops 10 untergebracht sind. Hierzu gehören in diesem Ausführungsbeispiel insbesondere zwei externe Kabelzuführungen 46, 48, wie dies anhand der Schnittdarstellung in Fig. 3 erkennbar ist. Die Kabelzuführung 48 umfaßt dabei an ihrem in den Fig. 4 und 5 oben liegenden Ende ein Bildaufnahmemodul, das ein aufgenommenes optisches Bildsignal in ein elektrisches Bildsignal umwandelt.

Über die Kabelzuführung 46, die am unteren Ende 50 des Handgriffs 12 einen Steckkontakt 52 aufweist, kann dem Laryngoskop 10 ein Lichtsignal zugeführt werden. Die Kabelzuführung 48 dient demgegenüber dazu, das optische Bildsignal eines an der Bildeintrittsöffnung 40 aufgenommenen Bildes in Form des elektrischen Bildsignals an eine hier nicht dargestellte Bildwiedergabeeinheit zu übertragen.

In der Querschnittsdarstellung in Fig. 3 ist weiterhin erkennbar, daß die Kabelzuführung 46 sich im Inneren des Handgriffs 12 in drei einzelne Stränge 46a, 46b und 46c aufspaltet, die im Bereich der Kupplung 16 an die einzelnen Stränge des Lichtleiters 32 anschließen (siehe Fig. 2).

In der vergrößerten Querschnittsdarstellung der Fig. 4 und 5 sind nur die Stränge 32a und 32b des Lichtleiters 32 sowie der Strang 46a der Kabelzuführung 46 erkennbar. Der Strang 32a des Lichtleiters 32 weist ebenso wie die verbleibenden Stränge 32b, 32c an seinem unteren Ende 62, das hier nachfolgend auch als proximales Ende bezeichnet wird, eine Lichteintrittsöffnung 64 auf. Der Lichteintrittsöffnung 64 gegenüber befindet sich in dem griffseitigen Teil 20 des Laryngoskops 10 die Lichtaustrittsöffnung 66 des Strangs 46a der Kabelzuführung 46. Wenn der Spatel 14 mit dem Handgriff 12 fest verbunden ist, wie dies in Fig. 4 dargestellt ist, liegen die Lichteintrittsöffnung 64 und die Lichtaustrittsöffnung 66 unmittelbar einander gegenüber.

In vergleichbarer Weise besitzt der Bildleiter 34 an seinem proximalen Ende 62 eine Bildaustrittsöffnung 68, die einer Bildeintrittsöffnung 70 des Bildaufnahmemoduls 49 gegenüber liegt. Die genannten Eintritts- und Austrittsöffnungen 64 bis 70 sind jeweils durch ein Deckglas verschlossen, um das Eindringen von Schmutz oder Staubteilchen zu verhindern.

Wie insbesondere in der Darstellung in Fig. 5 erkennbar ist, sind die Lichtaustrittsöffnung 66 und die Bildeintrittsöffnung 70 in zwei unterschiedlichen Koppelebenen 72, 74 angeordnet, die in Richtung der Längsmittelachse 76 des Handgriffs 12 axial zueinander verschoben sind. Hierdurch wird erreicht, daß ein im Bereich der Lichtaustrittsöffnung 66 entstehendes Streulicht nicht unmittelbar durch die Bildeintrittsöffnung 70 in den Bildtrakt einstreuen kann. In entsprechender Weise sind beim vorliegenden Ausführungsbeispiel auch die Lichteintrittsöffnung 64 sowie die Bildaustrittsöffnung 68 in unterschiedlichen, axial zueinander verschobenen Ebenen angeordnet.

Der Lichtleiter 32 und der Bildleiter 34 bestehen beim vorliegenden Ausführungsbeispiel des Laryngoskops 10 ausschließlich aus optischen Faserbündeln. Insbesondere der Bildleiter 34 kann jedoch alternativ hierzu auch ein Linsensystem beinhalten.

Der mechanische Teil der Kupplung 16 beinhaltet neben der Haltemutter 42 vor allem einen Konus 82 am proximalen Ende 62 des Spatels 14 sowie einen entsprechenden Gegenkonus 84 am oberen Ende des Handgriffs 12. Des weiteren ist ein Orientierungsstift 86 vorgesehen, der beim Verbinden des Spatels 14 mit dem Handgriff 12 in eine entsprechende Bohrung 88 eingeführt werden muß. Der Orientierungsstift 86 gewährleistet, daß der Spatel 14 jeweils nur so auf den Handgriff 12 aufgesetzt werden kann, daß sich die einander zugeordneten Eintritts- und Austrittöffnungen 64 bis 70 paßgenau gegenüberstehen. In Kombination mit der Haltemutter 42 bilden die genannten Elemente ein Zentrierelement 90, das gewährleistet, daß die einander zugeordneten Eintrittsund Austrittsöffnungen 64 bis 70 mit einer Paßungenauigkeit von maximal 0,1 mm zueinander starr fixiert werden, und zwar sowohl in radialer Richtung (Pfeil 92) als auch in axialer Richtung (Pfeil 94).

In dem Bildaufnahmemodul 49 ist eine Vergrößerungsoptik 96 enthalten, die das durch die Bildeintrittsöffnung 70 eintretende, optische Bildsignal etwa um den Faktor 1:5 vergrößert und anschließend einem hier nicht dargestellten elektronischen Bildaufnehmer zuführt. Der elektronische Bildaufnehmer ist im vorliegenden Fall ein CCD-Chip, der das optische Bildsignal in das elektrische Bildsignal umwandelt. Es kann hier jedoch auch jeder andere elektronische Bildaufnehmer verwendet werden. Der CCD-Chip beinhaltet gemäß einer besonders bevorzugten Ausführung der Erfindung zudem auch Mittel, um in Ergänzung zu dem mechanischen Zentrierelement 90 auch eine elektronische Bildzentrierung durchzuführen. Dies wird insbesondere dadurch realisiert, daß der CCD-Chip eine größere aktive Fläche aufweist, als benötigt, so daß mit Hilfe an sich bekannter Maßnahmen der Bildverarbeitung der relevante Bildausschnitt auch dann extrahiert werden kann, wenn die Bildaustrittsöffnung 68 und die Bildeintrittsöffnung 70 aufgrund einer Krafteinwirkung auf das Laryngoskop 10 nicht optimal zueinander positioniert sind.

Bei der nachfolgenden Beschreibung der Ausführungsbeispiele der Erfindung bezeichnen gleiche Bezugszeichen dieselben Elemente wie in den Fig. 1 bis 5.

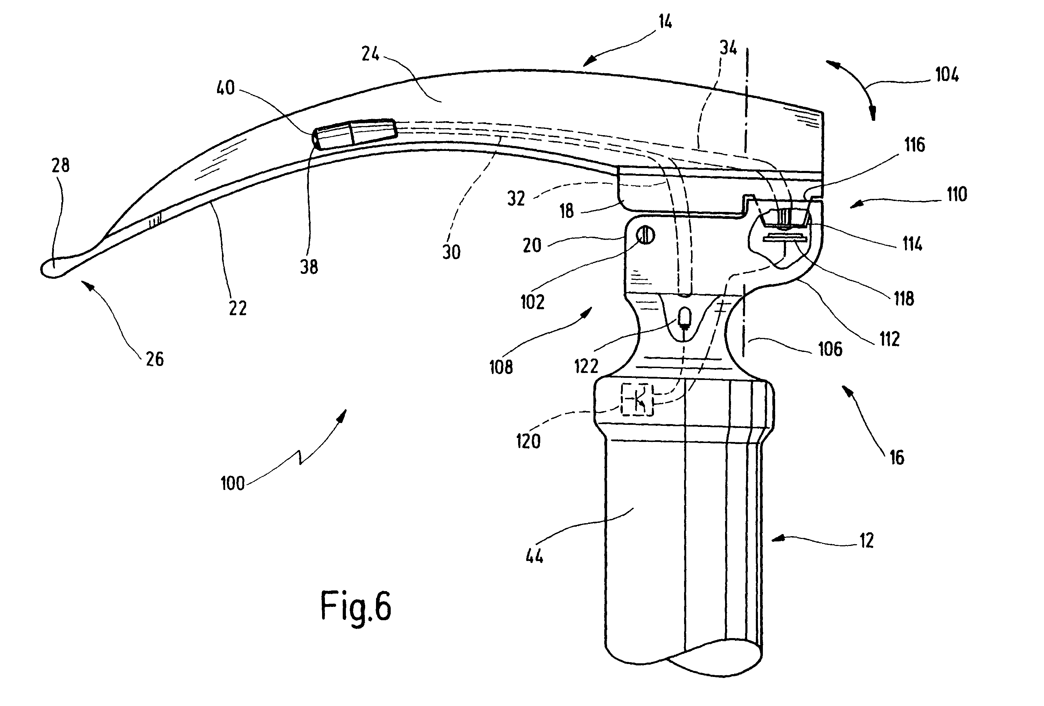

In Fig. 6 ist ein Ausführungsbeispiel eines erfindungsgemäßen Laryngoskops insgesamt mit der Bezugsziffer 100 bezeichnet.

Das Laryngoskop 100 unterscheidet sich von dem Laryngoskop 10 vor allem dadurch, daß die Kupplung 16 hier eine Standardkupplung nach der internationalen Norm ISO 7376-3 ist. Diese Art von Kupplung weist einen Querstift 102 auf, der an dem griffseitigen Teil 20 der Kupplung 16 angeordnet ist. Der spatelseitige Teil 18 besitzt eine in dieser Darstellung nicht erkennbare, U-förmige Ausnehmung, mit der der Spatel 14 auf den Querstift 102 aufgesetzt werden kann. Dabei kann der Spatel 14 in Richtung des Pfeils 104 um den Querstift 102 verschwenkt werden, was das Zusammenfügen und Auseinandernehmen des Laryngoskops 100 erleichtert.

Um eine unbeabsichtigte Schwenkbewegung in Richtung des Pfeils 104 beim Einsatz des Laryngoskops 100 zu verhindern, sind im Innern der Kupplung 16 hier nicht erkennbare Rastelemente vorhanden.

Die Norm ISO 7376-3 definiert nur denjenigen Teil der Kupplung 16, der links von der Linie 106 in Fig. 6 liegt. Dieser Kupplungsbereich wird nachfolgend mit der Bezugsziffer 108 bezeichnet. Rechts von der Linie 106 befindet sich bei diesem Ausführungsbeispiel ein zusätzlicher Kupplungsbereich 110, durch den die Kontaktfläche der Kupplung 16 gegenüber der Norm ISO 7376-3 erweitert wird. Der zusätzliche Kupplungsbereich 110 ist entsprechend einer bevorzugten Variante hier diametral gegenüber von dem distalen Ende 26 des Spatels 14 angeordnet.

In dem zusätzlichen Kupplungsbereich 110 ist bei diesem Ausführungsbeispiel die Kopplung für den Bildleiter 34 angeordnet. Dies hat zur Folge, daß der Handgriff 12 des Laryngoskops 100 auch mit einem Standardspatel verbunden werden kann, der keinen Bildleiter aufweist, da dieser Standardspatel nur bis zu der Linie 106 heranreicht. Ebenso kann der hier dargestellt Spatel 14 mit dem Handgriff eines Standard-Laryngoskops verbunden werden, dessen griffseitiges Teil 20 den Kupplungsbereich 110 nicht aufweist.

Im vorliegenden Ausführungsbeispiel weist der Kupplungsbereich 110 am griffseitigen Teil 20 einen Flansch 112 auf, der an seinem oberen Ende einen Innenkonus 114 besitzt. Der spatelseitige Teil 18 besitzt einen entsprechenden Außenkonus 116. Der Innenkonus 114 und der Außenkonus 116 sind wie im vorhergehenden Ausführungsbeispiel Bestandteil eines Zentrierelements 90, das in Verbindung mit den hier nicht dargestellten Rastmitteln die Eintritts- und Austrittsöffnungen 64 bis 70 in ihrer vorbestimmten Position zueinander fixiert. Aus Gründen der Übersichtlichkeit sind die zuletzt genannten Bezugszeichen in der Darstellung der Fig. 6 nicht eingezeichnet.

Sofern die bei der Standardkupplung 16 vorgesehenen Rastmittel nicht ausreichen, um die erforderliche Paßgenauigkeit starr zu gewährleisten, können weitere Rastmittel ergänzt werden, die bevorzugt ebenfalls in dem Kupplungsbereich 110 angeordnet werden. Bevorzugt wird dabei eine Haltemutter ähnlich derjenigen in Fig. 1 oder ein anderer, nur von Hand lösbarer Verriegelungsmechanismus verwendet.

In der teilweise aufgeschnittenen Darstellung des Kupplungsbereichs 110 ist ein elektronischer Bildaufnehmer 118 in Form eines CCD-Chips erkennbar. Dessen Signale sind einer Auswerteund Steuereinheit 120 zugeführt, die ebenfalls im Handgriff 12 des Laryngoskops 100 angeordnet ist. Des weiteren besitzt das Laryngoskop 100 im griffseitigen Teil 20 eine Lichtquelle 122, deren Licht unmittelbar in den Lichtleiter 32 eingekoppelt wird.

Im übrigen entspricht die Funktionsweise des Laryngoskops 100 derjenigen des Laryngoskops 10.

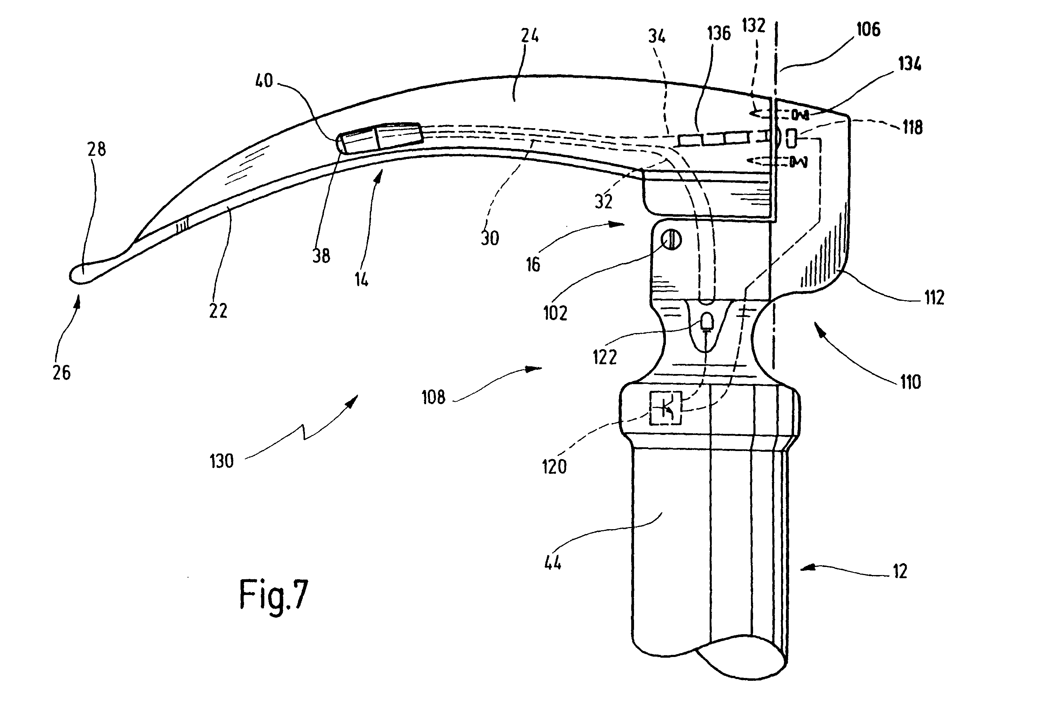

In dem Ausführungsbeispiel gemäß Fig. 7 ist ein erfindungsgemäßes Laryngoskop in seiner Gesamtheit mit der Bezugsziffer 130 bezeichnet.

Das Laryngoskop 130 unterscheidet sich von dem Laryngoskop 100 gemäß Fig. 6 im wesentlichen durch eine abweichende Erweiterung der Standardkupplung 16, die sich jedoch nur auf den zusätzlichen Kupplungsbereich 110 beschränkt. Derjenige Teil der Kupplung 16, der in Fig. 7 links von der Linie 106 liegt, entspricht in vollem Umfang der Norm ISO 7363-3.

Bei dem Laryngoskop 130 ist der elektronische Bildaufnehmer 118 in einem Flansch 112 angeordnet, der sich in Axialrichtung des Handgriffs 12 bis zum oberen, proximalen Ende des Spatels 14 erstreckt. Der Spatel 14 wird an dem Flansch 112 durch konische Paßstifte 132 zentriert, die in entsprechende konisch geformte Ausnehmungen an der seitlichen Wand 24 des Spatels 14 eingreifen. Die Paßstifte 132 sind auf Federn 134 gelagert, so daß ein Spatel, der die erforderlichen Ausnehmungen nicht aufweist, die Paßstifte 132 beiseite drücken kann. Hierdurch ist es möglich, auch bei dem Laryngoskop 130 einen normalen Standard-Spatel zu verwenden.

Aufgrund der Tatsache, daß bei dem Laryngoskop 130 der Bildleiter 34 weit weniger gekrümmt verläuft, ist es möglich, hier einen Bildleiter 34 zu verwenden, der ein Linsensystem 136 beinhaltet. Im übrigen entspricht die Funktionsweise des Laryngoskops 130 jedoch derjenigen des Laryngoskops 100.

Weitere Ausführungsbeispiele der Erfindung ergeben sich, wenn man Merkmale der hier exemplarisch dargestellten Ausführungsbeispiele untereinander kombiniert. So ist es beispielsweise möglich, das Laryngoskop 10 mit einer internen Lichtquelle 122 auszustatten. Umgekehrt können die Laryngoskope 100, 130 mit einer Kabelzuführung 46 versehen werden, über die das Licht einer Lichtquelle von außen zugeführt werden kann. Des weiteren können die Laryngoskope 100, 130 mit einer elektronischen Bildzentriereinheit ausgestattet sein.

In den weiteren Ausführungsbeispielen besitzen die gezeigten Laryngoskope keine Kabelzuführung 48, sondern statt dessen eine Bildwiedergabeeinheit, die direkt am Handgriff 12 des Laryngoskops befestigt bzw. sogar in diesen integriert ist. In diesem Fall ergibt sich ein vollständig autonomes Videolaryngoskop, das ohne externe Kabelzuführungen auskommt.

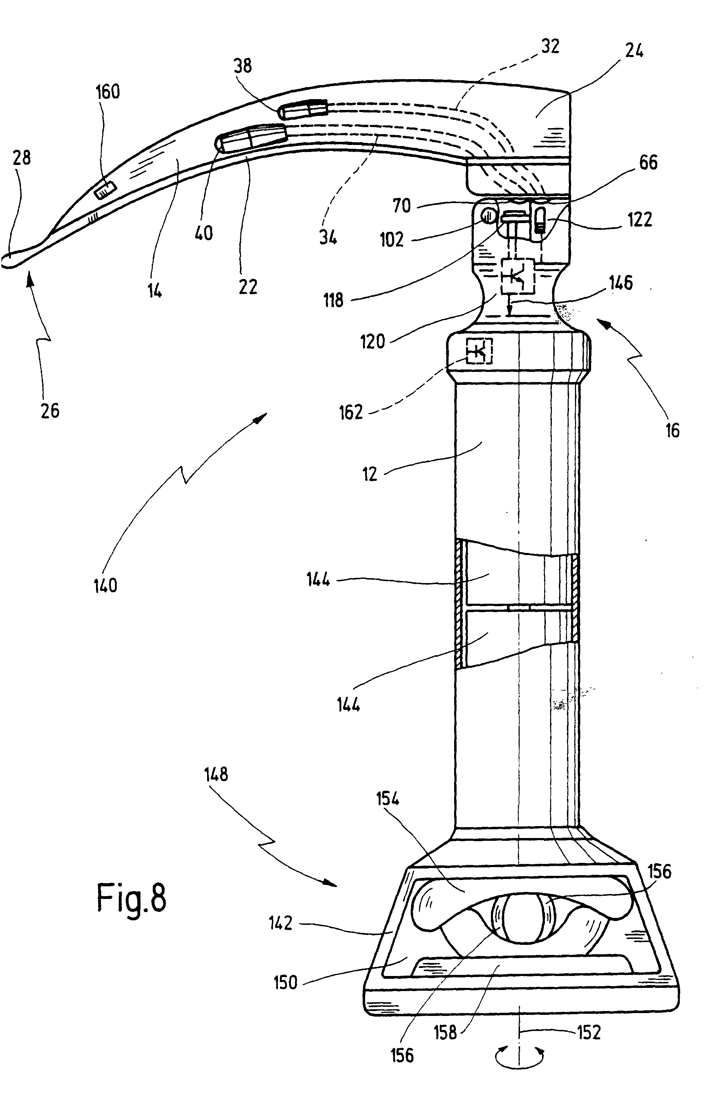

In Fig. 8 ist ein autonomes Videolaryngoskop in seiner Gesamtheit mit der Bezugsziffer 140 bezeichnet, das in dieser Form allerdings nicht unter den Schutz der Erfindung fällt, da es keinen Zusätzlichen Kupplungsbereich aufweist.

Zur elektrischen Versorgung der Bildaufnahmeeinheit 118, der Lichtquelle 122, der Auswerte- und Steuereinheit 120 sowie der nachfolgend beschriebenen Bildwiedergabeeinheit 142 sind in dem Handgriff 12 hier zwei Akkus 144 untergebracht. Die Akkus 144 sind mit den genannten Komponenten elektrisch verbunden, was durch den Pfeil 146 angedeutet ist. Die Akkus 144 sind hier induktiv, d.h. berührungslos von außen, aufladbar.

Am proximalen Ende 148 des Handgriffs 12 ist die Bildwiedergabeeinheit 142 integriert, die im wesentlichen einen für den behandelnden Arzt sichtbaren Bildschirm 150 beinhaltet. Der Bildschirm 150 ist in LCD-Technologie in Sandwichbauweise aufgebaut und farbig. Die gesamte Bildwiedergabeeinheit 142 ist um die Längsachse 152 des Handgriffs 12 drehbar, so daß der behandelnde Arzt die Position des Bildschirms 150 nach seinen Bedürfnissen einstellen kann.

Zur Verdeutlichung eines über den Bildleiter 34 und die Bildaufnahmeeinheit 118 aufgenommenen und über die Bildwiedergabeeinheit 142 wiedergegebenen Bildfeldes ist hier auf dem Bildschirm 150 schematisch der Kehlkopfbereich eines Patienten mit der Epiglottis 154 und den Stimmlippen 156 dargestellt. Darüber hinaus ist in dem hier gezeigten Bildausschnitt ein Abbild 158 des Wulstes 28 am distalen Ende 26 des Spatels 14 zu erkennen.

In dem hier dargestellten Ausführungsbeispiel ist die Bildwiedergabeeinheit 142 vom proximalen Ende 148 des Handgriffs 12 abnehmbar. Dies ist im vorliegenden Ausführungsbeispiel erforderlich, um die Akkus 144 auszutauschen und kann außerdem geschehen, wenn die Bildwiedergabeeinheit 142 nicht benötigt wird. Im Normalfall ist die Bildwiedergaabeeinheit 142 jedoch an dem Handgriff 12 befestigt.

Des weiteren besitzt das Laryngoskop dieses Ausführungsbeispiels außerdem einen an sich bekannten Gassensor 160, der am distalen Ende 26 des Spatels 14 angeordnet ist. Mit Hilfe des Gassensors 160 ist es möglich, den Sauerstoffgehalt und/oder den CO₂-Gehalt im Bereich des Rachenraums des Patienten zu bestimmen. Der Gassensor 160 ist mit einer im Handgriff 12 angeordneten Auswerteeinheit 162 verbunden.

Darüber hinaus kann das Laryngoskop 140 mit einem hier nicht dargestellten Spülsystem versehen sein, mit dem eine Flüssigkeit, bspw. eine NaCl-Lösung, über den Handgriff 12 zum distalen Ende 26 des Spatels 14 geführt werden kann, um die Enden des Lichtleiters 32 und des Bildleiters 34 auch während der Anwendung zu säubern.

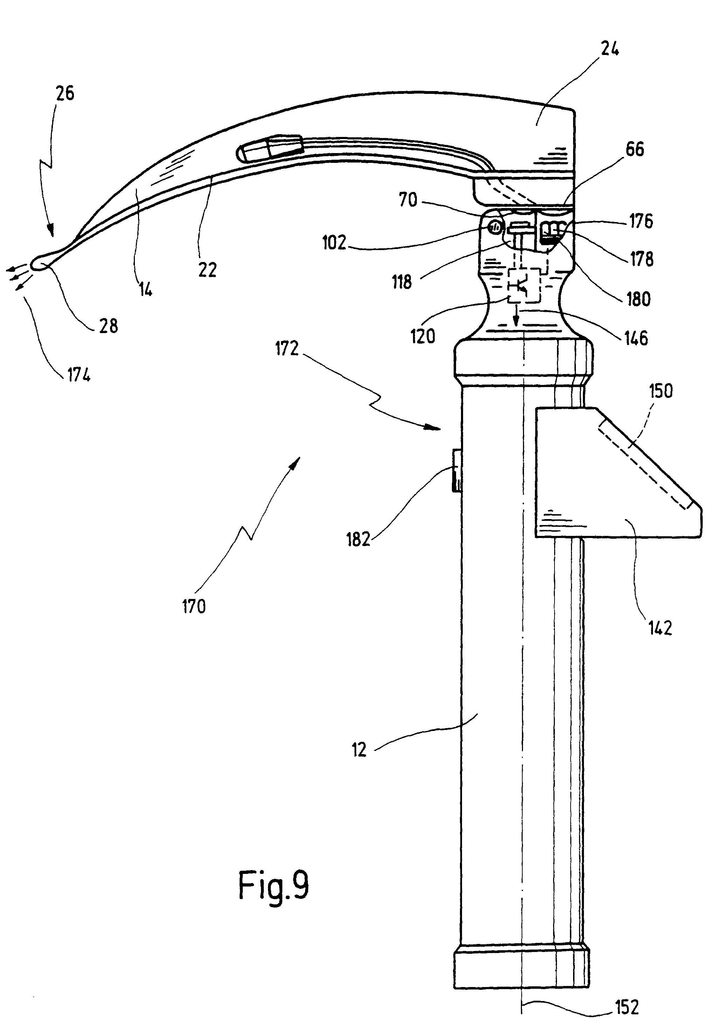

In Fig. 9 ist ein weiteres Ausführungsbeispiel eines autonomen Videolaryngoskops in seiner Gesamtheit mit der Bezugsziffer 170 bezeichnet.

Das Laryngoskop 170 unterscheidet sich von dem vorhergehenden Ausführungsbeispiel vor allem hinsichtlich der Bildwiedergabeeinheit 142, die hier am distalen Ende 172 des Handgriffs 12 starr angeordnet ist. Um den behandelnden Arzt eine gute Sicht auf den Bildschirm 150 zu ermöglichen, ist die Bildwiedergabeeinheit 142 auf derjenigen Seite des Handgriffs 12 angeordnet, die vom distalen Ende 26 des Spatels 14 abgewandt ist. In einer hier nicht dargestellten Variante dieses Ausführungsbeispiels ist der Bildschirm 150 bezüglich der Längsachse 152 des Handgriffs 12 kippbar.

Eine weitere Besonderheit des Laryngoskops 170 ist, daß der Spatel 14 aus einem transparenten, lichtleitenden Material, hier aus Plexiglas, besteht. Das Material ist so gewählt, daß das Spatelblatt 22 des Spatels 14 die Funktion des Lichtleiters übernimmt. Dementsprechend kommt das Laryngoskop 170 mit einer einzigen optischen Faser für den Bildleiter 34 aus. Wie in Fig. 9 durch Pfeile 174 angedeutet, wird das Licht zum distalen Ende 26 des Spatels 14 geführt und es tritt dort aus der Wulst 28 aus.

Ein weiterer Unterschied zum vorhergehenden Ausführungsbeispiel ist, daß das Laryngoskop 170 hier drei verschiedenfarbige Lichtquellen in Form von drei LEDs besitzt, die mit den Bezugsziffern 176, 178, 180 bezeichnet sind und die die Farben Rot, Grün und Blau aufweisen.

Des weiteren besitzt das Laryngoskop 170 an seinem Handgriff 12 eine Buchse 182 mit elektrischen Steckverbindungen für eine zusätzliche elektrische Versorgung von außen sowie zum Abgreifen der Signale, die der Bildwiedergabeeinheit 142 von der Bildaufnahmeeinheit 118 zugeführt werden. Hierdurch ist es möglich, das von der Bildaufnahmeeinheit 118 erzeugte Bild telemetrisch auch auf einem externen Monitor sichtbar zu machen. Dies kann sowohl in Ergänzung als auch alternativ zu einer Ausgabe auf dem Bildschirm 150 der Bildwiedergabeeinheit 142 geschehen. Darüber hinaus enthält die Buchse 182 Anschlüsse für eine externe Lichtquelle und ggf. für eine telemetrische Auswertung der Signale des Gassensors 160 des vorhergehenden Ausführungsbeispiels.

In einer besonderen Betriebsart des Laryngoskops 170 werden die Lichtquellen 176 bis 180 synchron zur Bildfrequenz der Bildaufnahmeeinheit 118 bzw. der Bildwiedergabeeinheit 142 gepulst, um den mittleren Energieverbrauch zu senken.

## Patentansprüche

1. Laryngoskop, mit einem Handgriff (12) und mit einem im wesentlichen quer dazu angeordneten Spatel (14), der über eine Kupplung (16) lösbar an dem Handgriff (12) befestigt ist, ferner mit einem Lichtleiter (32) und einem Bildleiter (34), die fest an dem Spatel (14) angeordnet sind, wobei der Lichtleiter (32) ein proximales Ende (62) mit einer Lichteintrittsöffnung (64) und wobei der Bildleiter (34) ein proximales Ende (62) mit einer Bildaustrittsöffnung (68) aufweist, wobei die Lichteintrittsöffnung (64) und die Bildaustrittsöffnung (68) im Bereich der Kupplung (16) angeordnet sind und wobei der Handgriff (12) im Bereich der Kupplung (16) eine Lichtaustrittsöffnung (66) sowie eine Bildeintrittsöffnung (70) aufweist, die ein Einkoppeln des Lichtsignals von dem Handgriff (12) in den Lichtleiter (32) sowie ein Auskoppeln des Bildsignals aus dem Bildleiter (34) ermöglichen, ferner mit einem Zentrierelement (90), das die Bildeintrittsöffnung (70) und die Bildaustrittsöffnung (68) paßgenau zueinander zentriert, **dadurch gekennzeichnet, daß** die Kupplung (16) eine Standardkupplung zum Verbinden von Laryngoskop-Spateln (14) mit Handgriffen (12) gemäß der internationalen Norm 7376-3 ist und daß die Kupplung (16) am proximalen Ende (62) des Bildleiters (34) einen zusätzlichen Kupplungsbereich (110) aufweist, durch den die Kontaktfläche der Kupplung (16) gegenüber der Norm ISO 7376-3 erweitert wird und in dem die Kopplung für den Bildleiter (34) angeordnet ist.

2. Laryngoskop nach Anspruch 1, **dadurch gekennzeichnet, daß** das Zentrierelement (90) die Bildeintrittsöffnung (70) und die Bildaustrittsöffnung (68) mechanisch zueinander zentriert.

3. Laryngoskop nach Anspruch 1 oder 2, **dadurch gekennzeichnet, daß** das Zentrierelement (90) die Bildeintrittsöffnung (70) und die Bildaustrittsöffnung (68) sowohl in radialer (92) als auch in axialer (94) Richtung zueinander zentriert.

4. Laryngoskop nach einem der Ansprüche 1 bis 3, **dadurch gekennzeichnet, daß** das Zentrierelement (90) die Eintrittsund Austrittsöffnungen (64 - 70) mit einer Paßungenauigkeit von weniger als 0,5 mm, vorzugsweise weniger als 0,1 mm, fixiert.

5. Laryngoskop nach einem der Ansprüche 1 bis 4, **dadurch gekennzeichnet, daß** das Zentrierelement (90) zumindest einen Konus (82; 114) sowie einen entsprechenden Gegenkonus (84; 116) aufweist, von denen einer an dem Handgriff (12) und der andere an dem Spatel (14) angeordnet ist.

6. Laryngoskop nach einem der Ansprüche 1 bis 5, **dadurch gekennzeichnet, daß** es einen elektronischen Bildaufnehmer (118), bevorzugt in Form eines CCD-Chips, sowie Mittel (120) zur elektronischen Bildzentrierung aufweist.

7. Laryngoskop nach einem der Ansprüche 1 bis 6, **dadurch gekennzeichnet, daß** die Bildeintrittsöffnung (70) und die Lichtaustrittsöffnung (66) in unterschiedlichen, axial zueinander versetzten Koppelebenen (72, 74) angeordnet sind.

8. Laryngoskop nach einem der Ansprüche 1 bis 7, **dadurch gekennzeichnet, daß** es ferner eine an dem Handgriff (12) angeordnete Bildwiedergabeeinheit (142) zum Wiedergeben eines aufgenommenen Bildes aufweist.

9. Laryngoskop nach Anspruch 8, **dadurch gekennzeichnet, daß** die Bildwiedergabeeinheit (142) auf einer von dem distalen Ende (26) des Spatels (14) abgewandten Seite des Handgriffs (12) angeordnet ist.

10. Laryngoskop nach Anspruch 8 oder 9, **dadurch gekennzeichnet, daß** die Bildwiedergabeeinheit (142) um eine Längsachse (152) des Handgriffs (12) drehbar ist.

11. Laryngoskop nach einem der Ansprüche 8 bis 10, **dadurch gekennzeichnet, daß** die Bildwiedergabeeinheit (142) in Bezug auf die Längsachse (152) des Handgriffs (12) kippbar ist.

12. Laryngoskop nach einem der Ansprüche 8 bis 11, **dadurch gekennzeichnet, daß** die Bildwiedergabeeinheit (142) von dem Handgriff (12) abnehmbar ist.

13. Laryngoskop nach einem der Ansprüche 1 bis 12, **dadurch gekennzeichnet, daß** zumindest ein Teil (22) des Spatels (14) aus einem lichtleitenden Material besteht, welches den Lichtleiter bildet.

14. Laryngoskop nach einem der Ansprüche 1 bis 13, **dadurch gekennzeichnet, daß** es mindestens zwei Bildleiter sowie zwei Bilderfassuhgseinheiten aufweist.

15. Laryngoskop nach einem der Ansprüche 1 bis 14, **dadurch gekennzeichnet, daß** am distalen Ende (26) des Spatels (14) ein Gassensor (160) zur Messung von Parametern eines Gasgemisches angeordnet ist.

16. Laryngoskop nach Anspruch 15, **dadurch gekennzeichnet, daß** der Gassensor (160) mit einer im Handgriff (14) angeordneten Auswerteeinheit (162) verbunden ist.

## Claims

1. A laryngoscope, comprising a handle (12) and a spattle (14), substantially transversely arranged thereto that is detachably fixed at the handle (12) via a coupling (16), further comprising an optical waveguide (32) and an image conductor (34) that are firmly arranged on the spattle (14), wherein the optical waveguide (32) comprises a proximal end (62) with a light inlet opening (64), and wherein the image conductor (34) comprises a proximal end (62) with an image outlet opening (68), wherein the light inlet opening (64) and the image outlet opening (68) are arranged in the region of the coupling (16), and wherein the handle (12) comprises, in the region of the coupling (16), a light outlet opening (66) and an image inlet opening (70) which allow the light signal to couple in from the handle (12) into the optical waveguide (32) as well as the image signal to couple out of the image conductor (34), further having a centering element (90) which centers the image inlet opening (70) and the image outlet opening (68) accurately fitting to each other, **characterized in that** the coupling (16) is a standard coupling according to International Standard ISO 7376-3 for connecting laryngoscope-spattles (14) with handles (12), and **in that** the coupling (16) comprises, at the proximal end (62) of the image conductor (34), an additional coupling region (110) with which the contact area of the coupling (16) is enlarged compared to standard ISO 7376-3 and in which the coupling for the image conductor (34) is arranged.

2. The laryngoscope of claim 1, **characterized in that** the centering element (90) mechanically centers the image inlet opening (70) and the image outlet opening (68) to each other.

3. The laryngoscope of claim 1 or 2, **characterized in that** the centering element (90) centers the image inlet opening (70) and the image outlet opening (68) both in radial (92) and in axial (94) direction to each other.

4. The laryngoscope of anyone of claims 1 through 3, **characterized in that** the centering element (90) fixes the inlet and outlet openings (64 - 70) with an inaccuracy in fitting of less than 0.5 mm, preferably less than 0.1 mm.

5. The laryngoscope of anyone of claims 1 through 4, **characterized in that** the centering element (90) comprises at least a cone (82; 114) as well as a corresponding opposed cone (84; 116), one of which being arranged at the handle (12) and the other one at the spattle (14).

6. The laryngoscope of anyone of claims 1 through 5, **characterized by** an electronic image pick-up unit (118), preferably in form of a CCD chip, and means (120) for electronic image centering.

7. The laryngoscope of anyone of claims 1 through 6, **characterized in that** the image inlet opening (70) and the light outlet opening (66) are arranged in different coupling planes (72, 74) that are axially displaced to each other.

8. The laryngoscope of anyone of claims 1 through 7, **characterized in that** it further comprises an image reproduction unit (142) arranged at the handle (12) in order to reproduce a picked up image.

9. The laryngoscope of claim 8, **characterized in that** the image reproduction unit (142) is arranged on a side of the handle (12) facing away from the distal end (26) of the spattle (14).

10. The laryngoscope of claim 8 or 9, **characterized in that** the image reproduction unit (142) is rotatable about a longitudinal axis (152) of the handle (12).

11. The laryngoscope of anyone of claims 8 through 10, **characterized in that** the image reproduction unit (142) can be tilted with reference to the longitudinal axis (152) of the handle (12).

12. The laryngoscope of anyone of claims 8 through 11, **characterized in that** the image reproduction unit (142) can be detached from the handle (12).

13. The laryngoscope of anyone of claims 1 through 12, **characterized in that** at least one part (22) of the spattle (14) consists of a light conducting material which forms the optical waveguide.

14. The laryngoscope of anyone of claims 1 through 13, **characterized in that** it comprises at least two image conductors as well as two image pick-up units.

15. The laryngoscope of anyone of claims 1 through 14, **characterized in that** a gas sensor (160) for measuring parameters of a gas mixture is arranged at the distal end (26) of the spattle (14).

16. The laryngoscope of claim 15, **characterized in that** the gas sensor (160) is connected to an evaluation unit (162) arranged in the handle (14).

## Revendications

1. Laryngoscope comportant une poignée (12) et une spatule (14) disposée sensiblement transversalement à celle-ci, laquelle est fixée, de manière séparable, à la poignée (12) par un accouplement (16), comportant en outre un guide de lumière (32) et un guide d'image (34), lesquels sont disposés de manière fixe sur la spatule (14), le guide de lumière (32) présentant une extrémité proximale (62) avec une ouverture d'entrée de lumière (64), et le guide d'image (34) présentant une extrémité proximale (62) avec une ouverture de sortie d'image (68), l'ouverture d'entrée de lumière (64) et l'ouverture de sortie d'image (68) étant disposées dans la zone de l'accouplement (16), et la poignée (12) présentant, dans la zone de l'accouplement (16), une ouverture de sortie de lumière (66) ainsi qu'une ouverture d'entrée d'image (70) qui permettent un couplage du signal de lumière de la poignée (12) dans le guide de lumière (32) ainsi qu'un découplage du signal d'image du guide d'image (34), comportant en outre un élément de centrage (90) qui centre de manière exactement ajustée l'une par rapport à l'autre l'ouverture d'entrée d'image (70) et l'ouverture de sortie d'image (68), **caractérisé en ce que** l'accouplement (16) est un accouplement standard pour relier des spatules de laryngoscope (14) à une poignée (12) selon la norme internationale 7376-3, et **en ce que** l'accouplement (16) présente, à l'extrémité proximale (62) du guide d'image (34), une zone d'accouplement (110) supplémentaire par laquelle la surface de contact de l'accouplement (16) est étendue par rapport à la norme ISO 7376-3 et dans laquelle est disposé l'accouplement pour le guide d'image (34).

2. Laryngoscope selon la revendication 1, **caractérisé en ce que** l'élément de centrage (90) centre mécaniquement l'une par rapport à l'autre l'ouverture d'entrée d'image (70) et l'ouverture de sortie d'image (68).

3. Laryngoscope selon la revendication 1 ou 2, **caractérisé en ce que** l'élément de centrage (90) centre l'une par rapport à l'autre l'ouverture d'entrée d'image (70) et l'ouverture de sortie d'image (68) aussi bien dans la direction radiale (92) que dans la direction axiale (94).

4. Laryngoscope selon l'une des revendications 1 à 3, **caractérisé en ce que** l'élément de centrage (90) fixe les ouvertures d'entrée et ouvertures de sortie (64-70) avec une précision d'ajustement inférieure à 0,5 mm, de préférence inférieure à 0,1 mm.

5. Laryngoscope selon l'une des revendications 1 à 4, **caractérisé en ce que** l'élément de centrage (90) comporte au moins un cône (82 ; 114) ainsi qu'un contre-cône (84 ; 116) correspondant dont un est disposé sur la poignée (12) et l'autre sur la spatule (14).

6. Laryngoscope selon l'une des revendications 1 à 5, **caractérisé en ce qu'**il comporte un dispositif électronique de prise de vues (118), de préférence sous la forme d'une puce CCD, ainsi que des moyens (120) pour le centrage électronique de l'image.

7. Laryngoscope selon l'une des revendications 1 à 6, **caractérisé en ce que** l'ouverture d'entrée d'image (70) et l'ouverture de sortie d'image (66) sont disposées dans des plans de couplage (72, 74) différents, décalés axialement l'un par rapport à l'autre.

8. Laryngoscope selon l'une des revendications 1 à 7, **caractérisé en ce qu'**il comporte en outre une unité de reproduction d'image (142) disposée sur la poignée (12) pour reproduire une image prise.

9. Laryngoscope selon la revendication 8, **caractérisé en ce que** l'unité de reproduction d'image (142) est disposée sur un côté de la poignée (12), tourné à l'opposé de l'extrémité distale (26) de la spatule (14).

10. Laryngoscope selon la revendication 8 ou 9, **caractérisé en ce que** l'unité de reproduction d'image (142) peut tourner autour d'un axe longitudinal (152) de la poignée (12).

11. Laryngoscope selon l'une des revendications 8 à 10, **caractérisé en ce que** l'unité de reproduction d'image (142) peut basculer par rapport à l'axe longitudinal (152) de la poignée (12).

12. Laryngoscope selon l'une des revendications 8 à 11, **caractérisé en ce que** l'unité de reproduction d'image (142) peut être retirée de la poignée (12).

13. Laryngoscope selon l'une des revendications 1 à 12, **caractérisé en ce qu'**au moins une partie (22) de la spatule (14) est constituée d'un matériau guidant la lumière qui forme le guide de lumière.

14. Laryngoscope selon l'une des revendications 1 à 13, **caractérisé en ce qu'**il comporte au moins deux guides d'image ainsi que deux unités d'enregistrement d'image.

15. Laryngoscope selon l'une des revendications 1 à 14, **caractérisé en ce qu'**à l'extrémité distale (26) de la spatule (14) est disposé un capteur de gaz (160) pour mesurer des paramètres d'un mélange de gaz.

16. Laryngoscope selon la revendication 15, **caractérisé en ce que** le capteur de gaz (160) est relié à une unité d'exploitation (162) disposée dans la poignée (14).
